Europäisches Patentamt

European Patent Office

Office européen des brevets

(19)

(11) Veröffentlichungsnummer: **0 277 540**
**A1**

(12) **EUROPÄISCHE PATENTANMELDUNG**

(21) Anmeldenummer: **88100709.0**

(22) Anmeldetag: **20.01.88**

(51) Int. Cl.⁴: **C12Q 1/34**

(30) Priorität: **23.01.87 DE 3701834**

(43) Veröffentlichungstag der Anmeldung:
**10.08.88 Patentblatt 88/32**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Bräu, Barbara, Dr.**
**Johannesallee 8**
**D-6230 Frankfurt am Main 80(DE)**
Erfinder: **Wöhner, Gerhard, Dr.**
**Flörsheimer Strasse 27**
**D-6093 Flörsheim am Main(DE)**
Erfinder: **Seibert, Gerhard, Prof. Dr.**
**Gläserweg 21**
**D-6100 Darmstadt(DE)**
Erfinder: **Then, Johann, Dr.**
**Sulzbacher Weg 2**
**D-6238 Hofheim am Taunus(DE)**

(54) **Verfahren zur Bestimmung der Lysozymaktivität.**

(57) N-Acetylmuramidase-Aktivität kann spezifisch mit Hilfe von Peptidoglycan, das aus grampositiven Bakterienzellen isoliert und mit einem Reaktivfarbstoff gefärbt wird, bestimmt werden.

EP 0 277 540 A1

## Verfahren zur Bestimmung der Lysozymaktivität

In dem U.S. Patent 3 834 991 wird eine kolorimetrische Bestimmungsmethode für Lysozym beschrieben. Man verwendet ganze Zellen von Micrococcus lysodeitkticus und behandelt sie mit einem Reaktivfarbstoff. Die gefärbten Zellen werden in wäßriger Lösung mit einer unbestimmten Menge Lysozym inkubiert. Aufgrund der Zelllyse löst sich der gebundene Farbstoff in der wäßrigen Lösung. Die Färbung der Lösung ist ein ungefähres Maß für die Lysozym-Konzentration in der Probe.

Der Vorgang der Zelllyse ist komplex und stellt lediglich ein indirektes Maß für die Lysozymaktivität dar [N.Grossowicz et al., In: Methods of Biochemical Analysis 29, 435 (1983)]. Es hat sich nun herausgestellt, daß der Test nicht spezifisch für Lysozym ist. Bei der Verwendung ganzer Zellen, bindet der Farbstoff grundsätzlich an freie $NH_2$- oder OH-Gruppen von z.B. Proteinen und Polysacchariden allgemein und Teichonsäuren der Zelloberfläche. Daraus folgt, daß beispielsweise in proteasehaltigen Lysozymproben auch die Proteaseaktivität mitgemessen wird, da Proteine abgebaut werden. Man erhält also eine verfälschte Aussage über die Lysozymkonzentration.

Die vorliegende Erfindung stellt daher eine Methode dar, mit der diese Nachteile überwunden werden können. Wenn isolierte Zellwände von grampositiven Bakterien für diesen Test eingesetzt werden, so kann man aufgrund der Färbung der Testlösung die genaue N-Acetylmuramidase Aktivität einer Probe errechnen.

Die Erfindung betrifft somit ein Verfahren zur Bestimmung der N-Acetylmuramidase Aktivität in einer Probe mit Hilfe von Reaktivfarbstoffen, dadurch gekennzeichnet, daß man isoliertes, gefärbtes Peptidoglycan von grampositiven Bakterien als Substrat verwendet.

Im folgenden wird die Erfindung im einzelnen, insbesondere in ihren bevorzugten Ausführungsformen, beschrieben. Ferner wird die Erfindung in den Ansprüchen definiert.

Mit der erfindungsgemäßen Testmethode kann die N-Acetylmuramidase-Aktivität grundsätzlich aller entsprechenden Enzymprodukte bestimmt werden. Bevorzugt wird sie jedoch zur Bestimmung von Lysozym, insbesondere in Körperflüssigkeiten, z. B. humanen Körperflüssigkeiten, und mureinlysierenden Enzymprodukten von Bakterien, insbesondere von Streptomyceten, eingesetzt.

Für den ·Test verwendet man Peptidoglycan von grampositiven Bakterien, bevorzugt jedoch von Micrococcus lysodeikticus. Das Peptidoglycan, auch Murein genannt, gewinnt man nach an sich bekannten Methoden durch Behandlung der Zellen mit Trichloressigsäure und einer Protease und anschließender Gewinnung des von Teichonsäuren, Proteinen und Polysacchariden weitgehend befreiten Murein. Der Begriff "weitgehend befreit" bedeutet, daß das Peptidoglycan mindestens so weit von den genannten Stoffen gereinigt sein muß, daß man bei Anwendung einer mit entsprechenden abbauenden Enzymen verunreinigten N-Acetylmuramidase keine verfälschte Aussage über die N-Acetylmuramidase-Aktivität erhält.

Das isolierte Peptidoglycan kann mit Reaktivfarbstoffen angefärbt werden, wie sie beispielsweise in den U.S. Patenten 3 834 991, 2 820 785, 2 889 316, 2 891 941, 2 892 828, 2 979 498, 3 054 795, 3 036 058, 3 149 100 oder 3 127 232 beschrieben sind. Es handelt sich im wesentlichen um Reaktivfarbstoffe, wie sie zum Anfärben von cellulosehaltigen Textilien verwendet werden. Bevorzugt werden die Farbstoffe Reactive Blue 19 (C.I. Nr. 612000), C.I. Reactive Green 14 (®Remazol Brilliant Grün GGL) sowie ®Cibacron Grün 4G für die Testmethode verwendet.

Die Färbung kann auf unterschiedliche Weise durch Mischen des Peptidoglycan mit dem Farbstoff in einem geeigneten Reaktionsmedium, beispielsweise wäßriges Alkalihydroxyd oder -carbonat bzw. verdünnte Säuren, wie z.B. Ameisensäure oder Essigsäure, durchgeführt werden. Das Verhältnis, in dem Peptidoglycan und Farbstoffgemisch eingesetzt werden, kann in weiten Bereichen schwanken, bevorzugt werden die beiden Komponenten jedoch in einem Bereich von 10:1 bis 1:10, bevorzugt 1:1 bis 1:3 miteinander gemischt. Das Reaktionsvolumen kann 25 bis 100 ml pro g Murein betragen, bevorzugt 50 bis 100 ml. Die Reaktionszeit und Temperatur sind ebenfalls nicht genau festgelegt. Vorteilhaft inkubiert man das Reaktionsgemisch über einen Zeitraum von 1 bis 20 Stunden bei einer Temperatur von 20 bis 100°C. Bevorzugt ist jedoch eine Inkubation von 5 Minuten bei 90°C bis 100°C und anschließend 15 bis 20 Stunden bei 20°C. Nach beendeter Reaktion wird der nicht verbrauchte Farbstoff mit Puffern von pH 3 bis 9, wie z.B. Natriumacetat oder -phosphat und organischen Lösungsmitteln, wie beispielsweise Ethanol oder Ether, abgewaschen. Nach der Trocknung kann das gefärbte Peptidoglycan dann dür den Test verwendet werden.

Die Bestimmung der Lysozymaktivität geschieht durch Inkubation des gefärbten Murein mit der N-Acetylmuramidase-haltigen Probe. Es sollte beispielsweise 1 bis 2 mg/ml Murein mit ca. 1 bis 10 mg/l (35 bis 350 U/ml) N-Acetylmuramidase inkubiert werden.

Die Reaktionstemperatur und Reaktionszeit kann auch hier wieder in weiten Bereichen - schwanken. Vorteilhaft arbeitet man bei Temperaturen zwischen 20 und 37°C und inkubiert unter leichtem Schütteln ca. 15 bis 60 Minuten. Insbesondere bevorzugt ist es die Reaktion nach 30 Minuten bei 25°C zu beenden. Dies kann durch Zugabe von beispielsweise Alkalihydroxyd [bei Verwendung von z.B. Reactive Blue 19 (C.I. Nr. 61200)] oder Salzsäure [bei Verwendung von z.B. ®Cibacron Grün 4G oder ®Remazol-Brillant Grün GGL] geschehen. Man trennt das unlösliche Peptidoglycan durch Zentrifugieren ab und mißt die Extinktion bei einer für den jeweiligen Farbstoff geeigneten Wellenlänge. Die erhaltenen Extinktionswerte sind der N-Acetylmuramidase-Aktivität direkt proportional.

In den folgenden Beispielen wird die Erfindung weiter im Detail erläutert. Prozentangaben beziehen sich auf das Gewicht, wenn nicht anders angegeben.

Beispiele

1. Isolierung von Murein aus Zellwänden von Micrococcus lysodeikticus

Die im folgenden aufgeführte Methode ist eine Modifikation des Verfahrens von Schleifer et al., Bacteriol. Rev. 36, 407 (1972)

18 g lyophilisierte Zellen von Micrococcus lysodeikticus (Sigma) werden in 10 l eisgekühltem destilliertem Wasser suspendiert. 2,5 l einer eiskalten 25 %igen wäßrigen Trichloressigsäurelösung werden zu dieser Suspension gegeben und 45 Minuten unter Eiskühlung inkubiert. Anschließend wird bei ca. 30 000 x g in einem Durchflußrotor (SS34, DuPont) zentrifugiert, das Zentrifugat mit 2 l destilliertem Wasser gewaschen und in 12,6 l 75 %(v/v) Ethanol wieder resuspendiert. Nach 15 Minuten Rühren bei Raumtemperatur wird wieder zentrifugiert, das Zentrifugat mit 2 l destilliertem Wasser gewaschen, in 9 l destilliertem Wasser wieder aufgenommen und bei 90°C in einem Wasserbad inkubiert. 2,5 l der 25 %igen wäßrigen Trichloressigsäure werden hinzugegeben und weitere 20 Minuten bei 90°C inkubiert. Die Mischung wird dann 15 Minuten in ein Eisbad gegeben und anschließend zentrifugiert. Das Sediment wäscht man 2 mal mit 2 l destilliertem Wasser und suspendiert in 3,2 l 0,05 molarem Ammoniumcarbonat-Puffer (pH 7,5, 37°C). Die Suspension wird mit 360 ml 1 mg/ml Trypsin in 0,05 molarem Ammoniumcarbonatpuffer (pH 7,5) versetzt, 2 Stunden bei 37°C inkubiert, zentrifugiert und 2mal mit 2 l eiskaltem destilliertem Wasser gewaschen. Das Pellet wird in 150 ml destilliertem Wasser suspendiert und lyophilisiert. Man erhält 4 g des isolierten Murein.

2. Färbung des Murein

1 g des lyophilisierten Murein gemäß Beispiel 1 wird 5 Minuten in einem kochenden Wasserbad mit 50 ml einer Lösung von 2 % (w/v) Reactive Blue 19 (C.I. Nr. 61200) in 1· N wäßriger Natriumhydroxid-Lösung behandelt und über Nacht bei Raumtemperatur stehen gelassen. Das gefärbte Murein wird 10 Minuten bei 30 000 x g zentrifugiert. Der nicht gebundene Farbstoff wird von dem Sediment entfernt durch Waschen bei 35°C mit je 50 ml Wasser, 0,08 molarem Natriumacetatpuffer (pH 4,4) und ungefähr 8 mal mit 0,1 molarem Natriumacetatpuffer (pH 5), bis der nach der Zentrifugation verbleibende Überstand farblos ist. Nach jedem Waschen werden die Zellen bei 30 000 x g 10 Minuten zentrifugiert. Das gefärbte Murein wird schließlich mit je 50 ml Wasser, Ethanol und Ether gewaschen, und über Nacht bei Raumtemperatur getrocknet. Das getrocknete Murein wird in 50 ml destilliertem Wasser suspendiert, 2 Minuten mit einem Ultraturrax homogenisiert und anschließend lyophilisiert. Man erhält ein feines Pulver, das bei Raumtemperatur längere Zeit gelagert werden kann.

3. Quantitative spektrophotometrische Bestimmung von Lysozym

2 mg des gefärbten Murein werden in einem Zentrifugenröhrchen in 0,95 ml 0,1 molarem Natriumacetat (pH 5,0) suspendiert. 50 $\mu$l der lysozymhaltigen Probe (20 bis 200 mg/l, d.h. 700 bis 7000 U/ml) werden hinzugegeben und in einem Schüttelgerät bei 25°C genau 30 Minuten inkubiert. Die Reaktion wird durch Zugabe von 1 ml 1N wäßriger Natriumhydroxid-Lösung beendet. Der Ansatz wird 5 Minuten bei 13 000 x g zentrifugiert.

Die Extinktion des Überstands wird bei einer Wellenlänge von 591 nm bestimmt. Ein Blindwert ohne Lysozym und Standards mit einer bekannten Menge an Lysozym werden ebenfalls wie oben angegeben behandelt. Die Färbung des Überstands ist der Lysozym-Konzentration in der Probe direkt proportional. Die Konzentrationen können anhand der Standardwerte errechnet werden oder aus einer entsprechenden Eichkurve abgelesen werden.

4. Automatisierter spektrophotometrischer Lysozymtest

Für die routinemäßige Bestimmung einer Vielzahl von Proben kann der in Beispiel 3 beschriebene Lysozymtest automatisiert werden. Dazu wird eine Suspension von 2 mg/ml des gefärbten Murein in 0,1 molarem Natriumacetat (pH 5,0) hergestellt. Jeweils 150 µl dieser Suspension werden mit Hilfe einer automatisierten Pipette ("Cetus Propette" Systems, Fa. Cetus, Emeryville, USA) in die 96 Vertiefungen einer Mikrotiterplatte transferiert. Dazu werden 50 µl einer geeigneten verdünnten Lysozymprobe (4 - 40 ml/l, d.h. 140 - 1400 U/ml) gegeben. Die Inkubation der Platten erfolgt 30 Minuten bei 25°C in einem Schüttelgerät. Die Reaktion wird durch Zugabe von 100 µl 4N NaOH beendet. Nach dem Mischen wird 15 Minuten bei 500 x g zentrifugiert. Je 200 µl des Überstands werden in eine neue Mikrotiterplatte transferiert und die Extinktion bei 570 nm bestimmt. In jeder Mikrotiterplatte werden Standardlösungen mit bekannten Lysozymkonzentrationen in derselben Weise behandelt. Als Leerwert dient eine leere Platte. Die Färbung des Überstands der Proben ist direkt proportional zur vorhandenen Lysozymkonzentration. Aus den Eichwerten kann die jeweilige Lysozymkonzentration einer Probe ermittelt werden.

5. Aktivitätsbestimmung von bakterienlysierendem Enzymprodukt:

Zu 2,8 ml einer Suspension von 0,2 mg Micrococcus luteus ATCC 4698 (Boehringer Mannheim) pro ml 0,1 M Natriumacetatpuffer (pH 5,0) werden 0,2 ml bakterienlysierendes Enzymprodukt-haltige Proben pipettiert und bei 25°C die Abnahme der Trübung durch Messung der Extinktion bei 450 nm bestimmt. Als 1 U wird die Extinktionsabnahme von 0,001 Photometer-Skalaeinheiten pro Minute definiert.

**Ansprüche**

1. Verfahren zur Bestimmung der N-Acetylmuramidase-Aktivität mit Hilfe von Reaktivfarbstoffen, dadurch gekennzeichnet, daß man isoliertes, gefärbtes Peptidoglycan aus grampositiven Bakterien als Substrat verwendet.

2. Verfahren nach Anspruch 1, dadurch gekennzeichnet, daß man Peptidoglycan aus Micrococcus lysodeikticus verwendet.

3. Verfahren nach Anspruch 1 oder 2, dadurch gekennzeichnet, daß man Peptidoglycan, das mit Reactive Blue 19 (C.I. Nr. 61200), C.I. Reactive Green 14 (®Remazol Brillant Grün GGL) sowie ®Cibacron Grün 4G gefärbt ist, verwendet.

4. Verfahren nach einem oder mehreren der Ansprüche 1 bis 3, dadurch gekennzeichnet, daß das gefärbte Peptidoglycan 15 bis 60 Minuten bei 20 bis 37°C mit den N-Acetylmuramidasehaltigen Proben inkubiert wird.

5. Verfahren nach Anspruch 4, dadurch gekennzeichnet, daß 30 Minuten bei 25°C inkubiert wird.

6. Verfahren nach einem oder mehreren der Ansprüche 1 bis 5, dadurch gekennzeichnet, daß die Lysozym-Aktivität oder die N-Acetylmuramidase-Aktivität eines Streptomyceten-Enzymprodukts bestimmt wird.

7. Verfahren nach einem oder mehreren der Ansprüche 1 bis 6, dadurch gekennzeichnet, daß die Lysozym-Aktivität oder die N-Acetylmuramidase-Aktivität eines humanen Enzymprodukts bestimmt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | CHEMICAL ABSTRACTS, Band 91, 13.-20. August 1979, Seiten 250-251, Zusammenfassung Nr. 51643y, Columbus, Ohio, US; FU TAN UNIVERSITY: "Colorimetric determination of lysozyme", & FU TAN HSUEH PAO, TZU JAN K'O HSUEH 1978, (1), 66-73 * Zusammenfasung * --- | 1-3,7 | C 12 Q 1/34 |
| Y | CHEMICAL ABSTRACTS, Band 93, Nr. 23, 8. Dezember 1980, Seite 202, Zusammenfassung Nr. 217036w, Columbus, Ohio, US; H. MAEDA: "A new lysozyme assay based on fluorescence polarization or fluorescence intensity utilizing a fluorescent peptidoglycan substrate", & J. BIOCHEM. (TOKYO) 1980, 88(4), 1185-91 * Zusammenfassung * --- | 1-3,7 | |
| A | BIOLOGICAL ABSTRACTS, Band 68, Nr. 7, 1979, Seiten 3901-3902, Zusammenfassung Nr. 39072, Philadelphia, PA, US; G.A. TURNER et al.: "Evaluation of 3,4-dinitrophenyl tetra-N-acetyl-beta-chitotetraoside as a substrate for the measurement of lysozyme in normal and pathological sera", & ANN. CLIN. BIOCHEM. 16(1), 51-53, 1979 * Zusammenfassung * --- -/- | 1,7 | RECHERCHIERTE SACHGEBIETE (Int. Cl.4) C 12 Q |

Der vorliegende Recherchenbericht wurde für alle Patentansprüche erstellt

| Recherchenort | Abschlußdatum der Recherche | Prüfer |
|---|---|---|
| DEN HAAG | 26-04-1988 | HITCHEN C.E. |

Europäisches
Patentamt

## EUROPÄISCHER RECHERCHENBERICHT

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 102, Nr. 3, 21. Januar 1985, Seite 319, Zusammenfassung Nr. 20286a, Columbus, Ohio, US; J.F. BARRETT et al.: "The mechanism of soluble peptidoglycan hydrolysis by an autolytic muramidase. A processive exodisaccharidase", & J. BIOL. CHEM. 1984, 259(19), 11818-27 * Zusammenfassung * ----- | 1 | |

RECHERCHIERTE
SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 26-04-1988 | HITCHEN C.E. |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes
Dokument

EPO FORM 1503 03.82 (P0403)